# EUROPEAN PATENT APPLICATION

(11) **EP 3 756 636 A1**
(43) Date of publication of application: **30.12.2020**
(21) Application number: 18906044.5
(22) Date of filing: 06.09.2018
(51) Int. Cl.: A61H 1/02, A61F 5/01, A61F 5/058

(54) **THERAPEUTIC DEVICE FOR PERFORMING PASSIVE SHOULDER ROTATION EXERCISES**

(30) Priority: 19.02.2018 DO 2018000054
(71) Applicant: Gonzalez Ruiz, Guarocuya, Naco Santo Domingo, D.N., 10119 (DO)
(72) Inventor: Gonzalez Ruiz, Guarocuya, Naco Santo Domingo, D.N., 10119 (DO)
(74) Representative: Lorente Berges, Ana
(86) International application number: PCT/DO2018/050002
(87) International publication number: WO 2019/158176

(57) **Abstract**

The invention relates to a therapeutic device that surrounds and firmly grips the upper part of the arm and which allows the therapist to apply a pair of opposing tangential forces **(13)** to the protrusions or protuberances **(6)** and **(7)** of the device in order to perform passive internal and external shoulder rotation exercises without transmitting any force to the elbow. This means of applying forces directly to the upper part of the arm prevents the forearm from having to be used as a lever to perform rotations, which can damage or affect the elbow during the therapeutic process. In addition, the device allows the greatest possible force to be applied to the shoulder to maximize recovery. The alternative design of the device allows a variable speed motor to be coupled by incorporation of coupling parts **(14), (15).**

## Description

### TECHNICAL FIELD OF THE INVENTION.

The device is intended for use in rehabilitation or physiotherapy of the shoulder, specifically for passive exercise of internal and external rotation of the shoulder, especially when suffering from "frozen shoulder".

### DESCRIPTION OF PREVIOUS ART.

The shoulder articulation, the most mobile of the body, is formed by three bones: humerus, scapula and clavicle. The sphere of the head of the humerus is attached to the cavity of the scapula, all wrapped in strong connective tissue, forming the joint capsule of the shoulder.

This joint can suffer the condition of "Frozen Shoulder" which prevents movement and causes severe pain. This condition is also known as **"Adhesive Capsulitis"** and consists of a chronic inflammation of the connective tissue that covers the entire shoulder joint, forming adhesions and causing pain, stiffness and decreased mobility of the arm.

The causes that can lead to suffer from Frozen Shoulder are: diabetes, hormonal changes (menopause), thyroid issues, heart conditions, immobilizations after an injury or surgery. The treatment of the Frozen Shoulder includes intense and daily physiotherapy. One of the passive exercises administered is the internal and external rotation of the shoulder.

The existing manual method for physiotherapy of passive internal and external rotation of the shoulder consists of the following. First the patient is placed lying on a stretcher; the arm is placed perpendicular to the axis of the spine on the axis formed by both shoulders; then with the elbow flexed in 90 degrees and the forearm pointing to the ceiling. Now the therapist holds the elbow with one hand (as fixed point of the hinge), and with the other hand holds the forearm by the wrist and applies the force in the form of a lever. The force applied to the forearm as a lever is transmitted to the elbow, which then transmits it to the shoulder through the upper arm. This method generates strains on the elbow that can injure or hurt it unnecessarily. Possible injuries in the elbow could be avoided if the force could be applied on the same axis of the upper arm, using a device that allows a good grip and good transfer of forces.

Several patented technologies for passive shoulder rotation therapy consist of devices in which the patient's forearm is firmly attached to the device. The forces generated on the device are applied to the forearm, using it as a lever, similar to the manual method for passive exercise described previously. These forces applied to the forearm (used as a lever) are transmitted through the elbow and then the effort reaches the shoulder. These are the most relevant patents:
The patent US 2017/0224516 A1, published on August 10, 2017, is entitled "Shoulder Orthosis Including Flexion/Extension Device". The inventors are PETER BONUTTI; et.al. The invention shows a shoulder orthosis device placed on the patient's trunk. The device also holds the forearm that is placed at 90 degrees from the arm, where the mechanical force is applied to the forearm by turning it clockwise. The applied force is transmitted first to the elbow and then to shoulder to generate shoulder rotations. The main drawback of this technology is that the force can only be applied on the forearm, so the healthy elbow can be hurt or injured when receiving the efforts generated. In addition, it could happen that to avoid injure the elbow due to the efforts generated; the therapist doesn't apply the force with the necessary intensity to promote the improvement of the shoulder.

The patent KR 20140114081 (A), published on September 26, 2014, is entitled "Exercise Equipment For Frozen Shoulders". The Inventor is [KR] LEE DO YOUNG. The invention describes a mechanical device that forcibly makes a circular movement, in order to do exercises of shoulder rotation, arm elevation, and internal and external rotation of the arm for frozen shoulders. The main drawback of this technology is that the force can only be applied on the forearm, so the healthy elbow can be hurt or injured when receiving the efforts generated. In addition, it could happen that to avoid injure the elbow due to the efforts generated; the therapist doesn't apply the force with the necessary intensity to promote the improvement of the shoulder.

In the patent US 2010/0076354 A1, published on March 25, 2010; entitled "Shoulder Continuous Passive Motion Device"; Inventor ROBERT KELLY. The invention presents a device that mechanically slides the forearm holder forward and backward. This equipment provides therapy not related to shoulder rotation therapy.

The patent WO 2006/058442 A1, published on June 8, 2006; entitled "System And Method For A Cooperative Arm Therapy And Corresponding Rotation Module". The Inventors are NEF; RIENER; et.al. The invention shows a mechanical device capable of offering internal and external rotation of the shoulder. The device supports the patient's arm and forearm, and using motors the device gives the necessary movements of the therapy. The main drawback of this technology is that the force can only be applied on the forearm, so the healthy elbow can be hurt or injured when receiving the efforts generated. In addition, it could happen that to avoid injure the elbow due to the efforts generated; the therapist doesn't apply the force with the necessary intensity to promote the improvement of the shoulder.

### DESCRIPTION OF THE INVENTION.

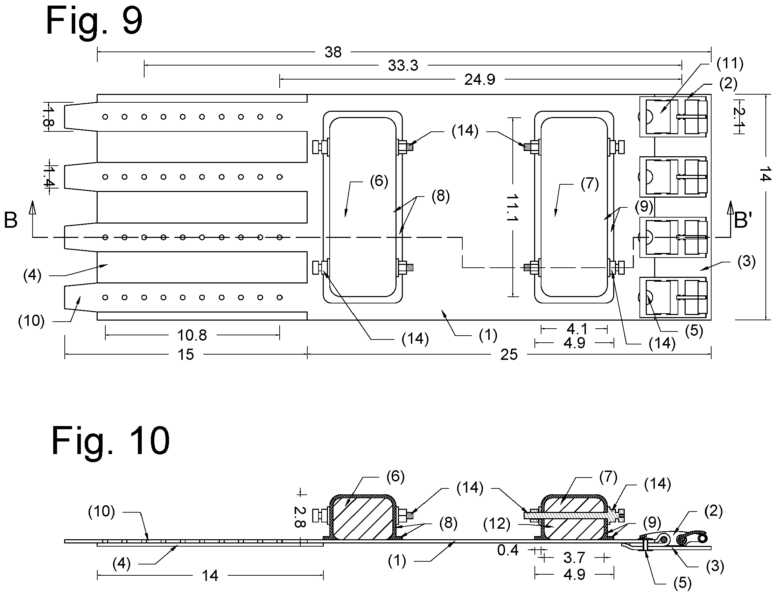

The "therapeutic device for performing passive shoulder rotation exercise" consists of an apparatus that allows the therapist to perform the passive exercise of internal and external rotation of the shoulder, applying the forces directly on the patient's upper arm; without the need for the forces to be applied on the forearm; and the efforts are not transmitted through the elbow; avoiding the elbow to be hurt or injured during the therapy. In the alternate design of the device **(****Figures 9, 10****,** **11****)** it can be coupled to a variable speed motor which is responsible for supplying the forces to perform the rotation of the shoulder.

All existing technologies apply forces on the forearm that are transmitted through the entire limb; the elbow receives unnecessary efforts that are unable to absorb. This situation can cause injuries to the elbow. It can also happen that to avoid injure the patient's elbow, due to the discomfort received, the forces are not applied with enough intensity to promote the improvement of the shoulder.

Using the proposed device, the necessary forces are applied on the upper arm for the internal and external shoulder rotation. The therapist can provide a stronger force that can be admitted by the shoulder to maximize shoulder recovery. This is done without the transmission of the efforts through the elbow that eliminates a possible injury of the elbow.

The proposed device is the first invention developed for passive exercise therapy of internal and external rotation of the shoulder that allows applying the necessary forces directly on the upper arm. The generated efforts are not transmitted through the elbow, avoiding hurting or injuring the elbow. In addition, the proposed device allows applying the maximum forces to the shoulder without being limited by possible injuries to the elbow.

The proposed device seeks to perform passive internal and external rotation of the shoulder, applying the necessary forces directly on the upper arm. The device avoids applying the forces through the forearm, avoiding injuries or discomfort on the elbow of the affected arm.

### BRIEF DESCRIPTION OF THE DRAWINGS.

- Figure 1.: Isometric of the patient using the device
- Figure 2.: Southeast isometric of the armed device
- Figure 3.: Southwest isometric of the armed device
- Figure 4A.: Front view of the armed device
- Figure 4B.: Frontal view of the device armed with the applied pair of forces.
- Figure 5.: Southeast isometric extended device.
- Figure 6.: Perspective view of the disarmed device
- Figure 7.: Top view of the dimensioned device
- Figure 8.: Section A-A' of the cut of the dimensioned device
- Figure 9.: Top view of the dimensioned device, including the coupling part to the motor.
- Figure 10.: Section B-B' of the dimensioned device, including the part of the motor coupling.
- Figure 11.: Front view of the armed device, including the part of the motor coupling and the torque applied.
- Figure 12.: Buckle: isometric, disassembled piece, top view, section C-C'.
- Figure 13.: Engine coupling part: Isometric, disassembled piece, side and front views.

### DETAILED DESCRIPTION OF THE INVENTION.

The "therapeutic device for performing passive shoulder rotation exercise" allows the forces to be applied directly on the upper arm, without stress being transmitted through the elbow. It avoids affecting or injuring the elbow, and also helps maximize the efforts received in the shoulder. The device wraps the upper arm, allowing it to be adjusted to the patient's arm and tightened enough by means of four straps **(10)** and their buckles **(5).** The closure prevents the device from sliding around the arm when applying the tangential forces **(13)** on the protrusions of the device **(6) (7).** The therapist applies the necessary forces on the protuberances to produce the internal or external rotation of the shoulder.

In the alternate design of the device **(****Figures 9, 10****,** **11****)** the tangential forces **(13)** are applied by a variable speed motor that can be attached to the device through the coupling part **(14) (15).**

The main material of the device needs to be flexible to adapt to the shape of the arm to wrap the patient's arm **(1) (3) (4) (10).** It must be resistant to traction to withstand the stresses to which it will be subjected and must create enough friction with the patient's arm to transmit the efforts generated during therapy. This material can be made of 2 mm thick leather or some synthetic material that emulates its properties. The coatings of the projecting parts where the tangential stress **(8) (9)** are applied are also of the same material.

Another element used in the construction of the device consists of rigid pieces with rounded edges **(6) (7).** The rigid pieces serve as a grip for the therapist to apply the pair of forces **(13).** The applied forces will be transmitted to the shoulder to perform the rotations. The material thereof can be made of wood or from a rigid plastic material. The rounded shape is used to preserve the covers **(8) (9),** and prevent them from breaking with use. Furthermore, metal buckles **(2)** are used in the construction of the device. The buckles are attached to the main piece **(1)** by wrapping the short strips **(11)** to one of the central axes of the buckles. They are fixed in place with the rivets **(5)** that hold the mentioned buckles **(2).** The rivets keep in place the leather protector **(3)** that separates the buckles from the arm.

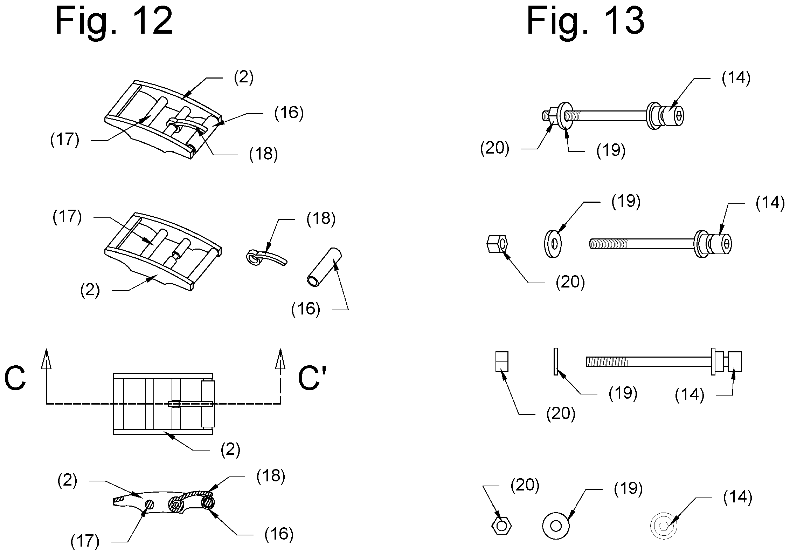

The buckles **(2)** can be seen in detail in **Figure 12** and are configured with a double central axis. The first axis contains the rotary nail **(18)** that engages and secures the belts **(10).** The other central axis **(17)** is embraced by the short strip **(11)** that joins the buckles **(2)** to the main piece **(1).** In addition, one of the external axes has a hollow cylinder **(16)** that freely rotates and facilitates the tightening of the belts **(10).**

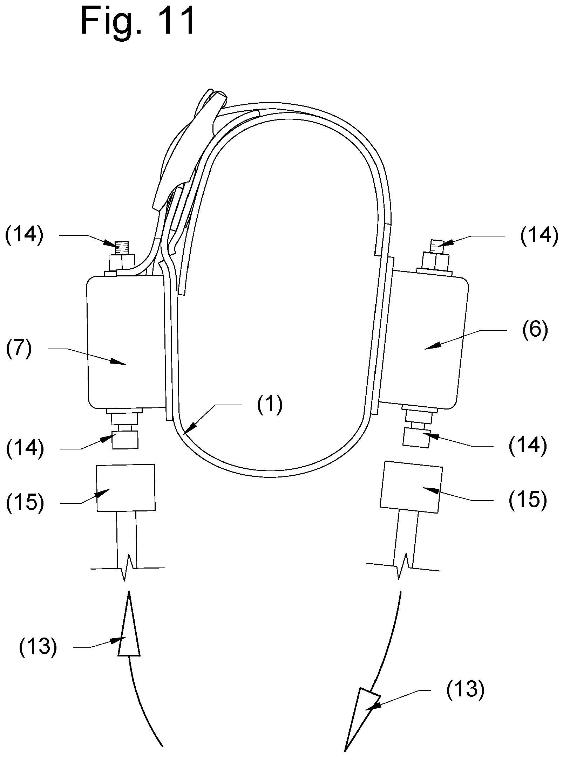

The **Figure 13** shows details of coupling parts **(14).** The **Figure 11** shows coupling **(14)** and the counterpart coupling **(15),** where both couplings connect the device to the variable speed motor. The coupling piece **(14)** is like a screw that passes through the grips of the device **(6) (7).** At one end the coupling piece consists of a nut **(20)** and a washer **(19),** while the opposite end has a hexagonal hole to use an Allen key. The coupling **(14)** in its main head has a cylindrical shape, whose axis in its central part reduces its diameter, serving the hollow formed for the coupling with the piece **(15);** and in the inner end of the head there is an integrated washer.

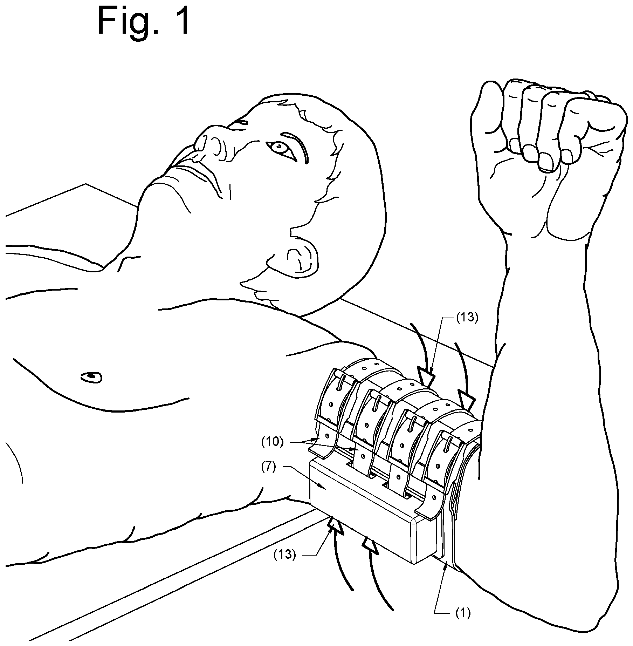

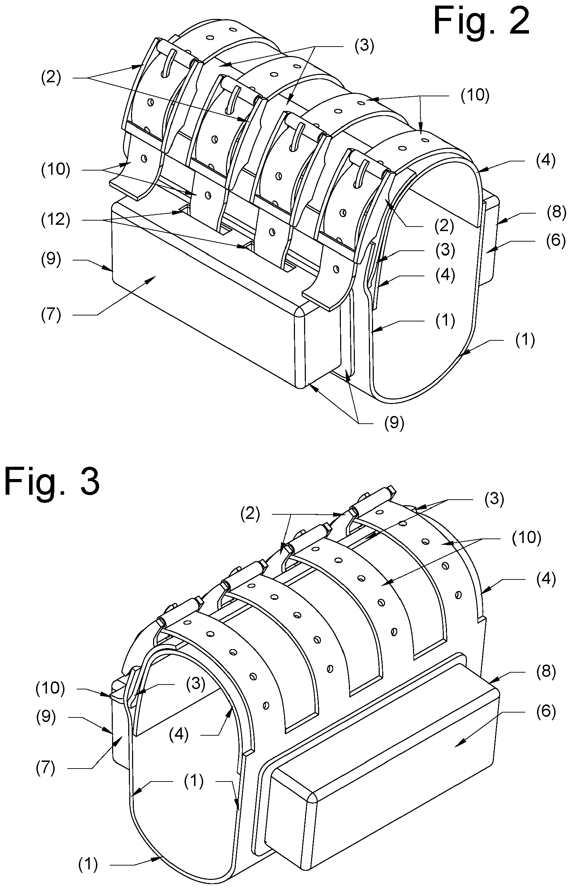

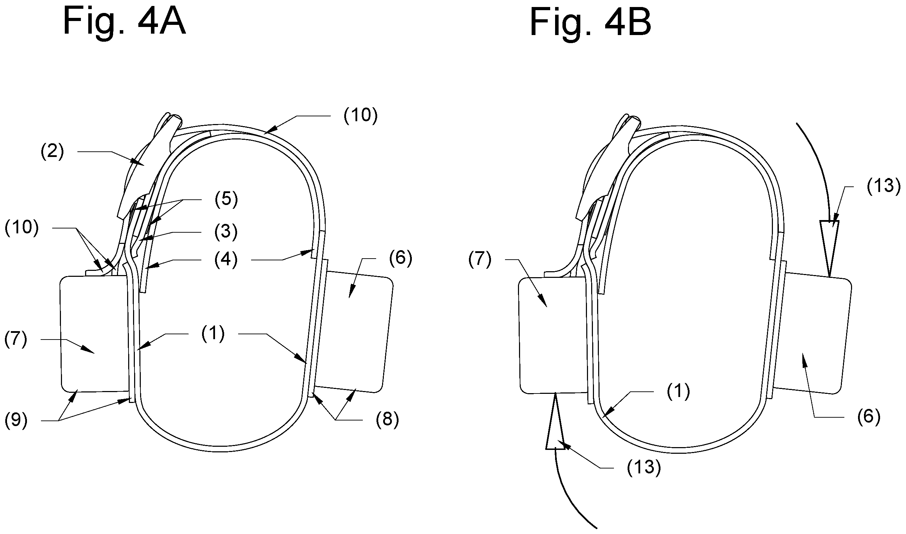

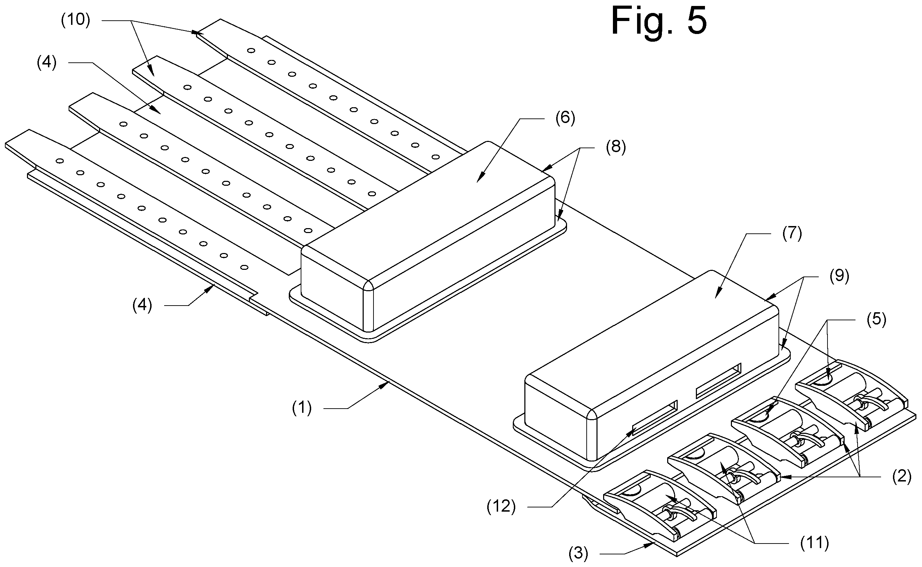

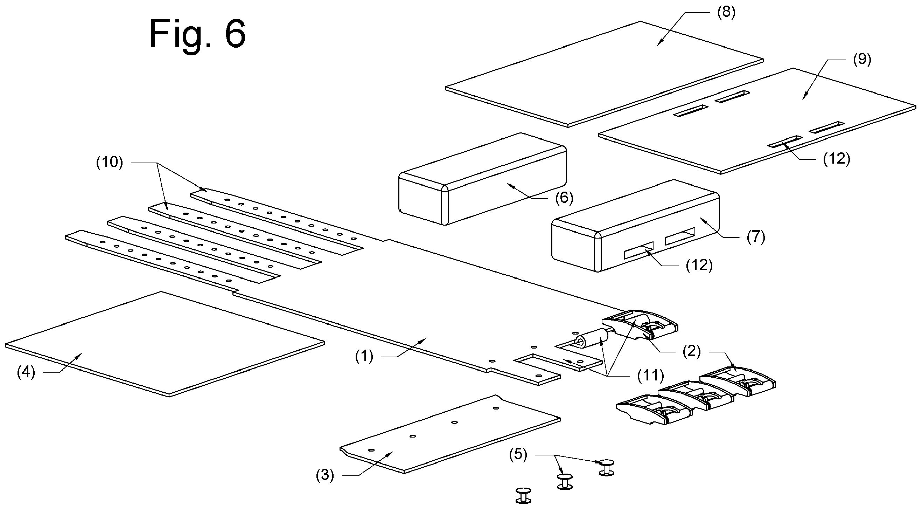

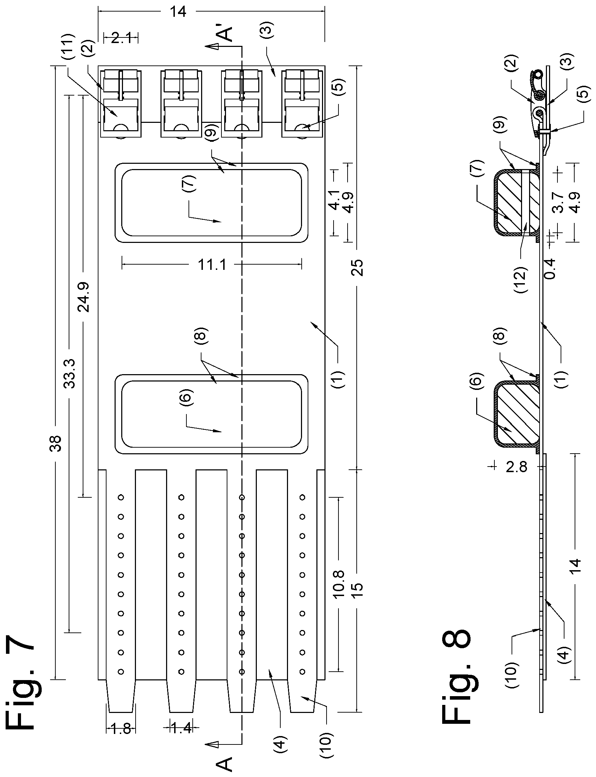

The device can be observed in **Figure 1** in a patient's arm, in **Figures 2** and **3** it is presented armed in southwest and southeast isometric views. **Figures 4A** and **4B** show a side view of the armed device, where **Figure 4B** adds the direction of the pair of forces applied **(13).** **Figure 5** shows a southeast isometric view of the extended device, **Figure 6** presents an isometric view with all the pieces of the device disassembled. **Figures 7** and **8** show the top view and the lateral section A-A' of the extended device, showing its main measurements. **Figures 9, 10** and **11** show the alternate design of the device, which incorporates the coupling parts **(14) (15)** so that the forces are provided by a variable speed motor. **Figures 12** and **13** present details of the buckle **(2)** and the coupling part **(14)** to the variable speed motor.

The dimensions of the device shown in **Figures 7 to 10** are designed for arms whose biceps tightened by the device are between 24.9 to 33.3 centimeters.

The device during its construction process is armed in the following way:
a) The main piece of the device **(1)** is leather (or other equivalent synthetic material), 2 mm thick, and wraps around the upper arm. It has at one end four straps or long strips **(10)** with multiple holes at short separation. On the other end it has four short strips **(11)** to which the metal buckles **(2)** will be fastened with the use of rivets **(5).** The long strips **(10)** enter the buckles **(2)** and tighten as much as possible to have a strong grip of the upper arm with the device, so when applying the tangential forces there is no slip between the arm and the device.
b) Several pieces of leather **(4) (3)** are incorporated to protect the patient's skin. It helps to improve the contact area between the leather and the arm. It also increase the friction force between the device and the arm, under the four long strips **(10)** and the four short strips **(11),** that will accommodate the buckles **(2).** Both pieces are sewn to the main piece **(1)** and also joined with glue.
c) The rivets used **(5)** secure the short strips **(11)** around one of the two central axes **(17)** of the buckles **(2).** The rivets also fix in place the protective piece **(3).**
d) Wood pieces with rounded edges **(6) (7)** or from another rigid plastic material are placed on top of the main leather piece **(1).** The wood pieces are fixed to it by means of leather wraps **(8) (9),** which are sewn to the main leather piece **(1),** leaving inside the pieces of wood. The piece of wood **(6)** is solid, while the other piece of wood **(7)** is crossed by two holes of rectangular entry **(12).** The holes will accommodate the excess of straps or long central strips **(10)** of the device when it is adjusted to the patient's arm, to avoid its interference in the grip of the piece by the therapist.

Next we describe the device when it is used by the therapist and when it is used with a variable speed motor:
- The first action is to place the device around the upper arm and tighten it firmly by using the straps **(10)** and the buckles **(2)** as shown in **Figure 1****;** then place the patient on the table with the upper arm on the same plane formed by the stretcher and perpendicular to the patient's column, with the forearm flexed and pointing to the ceiling. The therapist grasps with each hand one of the protuberances of the device **(6) (7).** Then the therapist exerts a pair of tangential and opposite forces, as indicated by the arrows **(13)** in **Figure 4B****,** and generates the internal or external rotation on the patient's shoulder.
- The alternating design of the device modifies the projections **(6) (7)** to incorporate the coupling part **(14)** that allows connecting the device with a variable speed motor. The motor exerts the pair of tangential and opposite forces that will produce the rotation of the shoulder. In addition, the wood piece **(7)** is solid and does not incorporate the holes **(12).**

## Claims

1. Therapeutic device for performing passive shoulder rotation exercise, **which includes:**
- a main piece **(1),**
- the protective pieces **(3)** (**4),**
- the projecting pieces **(6)** (**7),**
- the covers **(8)** (**9),**
- the holes **(12)** incorporated in the projecting pieces, and
- the coupling parts (14) to the variable speed motor.

2. Therapeutic device of Claim 1, **characterized in that** the main piece **(1)** wraps and compresses the upper arm, which is obtained through the use of straps **(10)** and buckles **(2).**

3. Therapeutic device of Claim 1, **characterized in that** the main piece **(1)** serves to support and transmit tensile and compressive stresses generated during the application of forces **(13).**

4. Therapeutic device of Claim 1, **characterized in that** the main piece **(1),** when pressed, creates friction between the device and the patient's skin.

5. Therapeutic device of Claim 1, **characterized in that** the use of protective pieces **(3)** (**4)** serve to protect the patient's skin, reducing and distributing the compression and shear forces generated.

6. Therapeutic device of Claim 1, **characterized in that** the protective pieces **(3) (4)** serve to support and transmit the tensile and compressive stresses generated during the application of the tangential forces **(13).**

7. Therapeutic device of Claim 1, **characterized in that** the protective pieces **(3) (4)** serve to increase the friction between the device and the skin of the patient's arm, preventing the device from sliding on the arm when applying tangential forces.

8. Therapeutic device of Claim 1, **characterized in that** the projecting pieces **(6) (7)** homogeneously transmit the applied tangential forces **(13)** as tensile and compressive stresses on the cross section of the device in its main part **(1)** and on the protectors **(3)** (**4).**

9. Therapeutic device of Claim 1, **characterized in that** the covers **(8) (9),** after being sewn to the main piece **(1),** containing the projecting pieces **(6)** (**7)**, allow the therapist to offer a non-slip grip so that they do not slip from their hand when applying tangential forces **(13).**

10. Therapeutic device of Claim 1, **characterized in that** the holes **(12),** made on the projecting part **(7)** and its cover **(9),** allow the surpluses of the belts **(10)** after being tightened to enter into the holes **(12)** to prevent them from interfering with the therapist's grip on the projecting piece **(7).**

11. Therapeutic device of Claim 1, **characterized in that** the coupling parts **(14),** which are incorporated into the projecting pieces **(6)** (**7),** allow the device to be connected to a variable speed motor.
